# EUROPEAN PATENT SPECIFICATION

(11) **EP 2 428 258 B1**
(45) Date of publication and mention of the grant of the patent: **29.03.2017**
(21) Application number: 10850414.3
(22) Date of filing: 20.07.2010
(51) Int. Cl.: A61K 36/00, B01D 11/02

(54) **PROCESS FOR EXTRACTING PLANT-DERIVED NATURAL PRODUCTS WITH POLARITY OR INTERMEDIATE POLARITY**
VORRICHTUNG ZUR EXTRAKTION VON AUS PFLANZEN GEWONNENEN NATÜRLICHEN PRODUKTEN MIT POLARITÄT ODER ZWISCHENPOLARITÄT
PROCÉDÉ POUR L'EXTRACTION DE PRODUITS NATURELS ISSUS DE PLANTES PRÉSENTANT UNE POLARITÉ OU UNE POLARITÉ INTERMÉDIAIRE

(30) Priority: 31.05.2010 CN 201010195507
(43) Date of publication of application: 14.03.2012
(73) Proprietor: China Agricultural University, Beijing 100083 (CN); Chenguang Biotech Group Co., Ltd., Hebei 057250 (CN)
(72) Inventor: LIAO, Xiaojun, Beijing 100083 (CN); LU, Qingguo, Hebei 057250 (CN); XU, Zhenzhen, Beijing 100083 (CN); LIAN, Yunhe, Hebei 057250 (CN)
(74) Representative: V.O.
(86) International application number: PCT/CN2010/001091
(87) International publication number: WO 2011/150536

(56) References cited:
- EP-A1- 0 917 876
- WO-A1-2008/026446
- CN-A- 1 172 669
- CN-A- 1 228 432
- CN-A- 1 931 259
- JP-A- 5 310 602
- US-A- 3 879 569
- US-B1- 6 326 504

## Description

### Technical Field

The present invention relates to a method for extracting natural products, in particular, a method for extracting natural products with High Pressure Carbon Dioxide (HPCD).

### Background Art

Generally, natural products are mostly extracted with solvent extraction method, the method comprises steps of selecting a solvent in which the target extracts have higher solubility and the non-target extracts have lower solubility or insolubility based on the solubility of the target extracts in the solvent, so as to selectively allow the target extracts to dissolve out from the matrix and enter into the solvent. Solvent extraction method includes water extraction and organic solvent extraction, since the target extracts can be generally divided into hydrophilic and lipophilic compounds, the organic solvent extraction can be further divided into hydrophilic organic solvent (such as ethanol, methanol, acetone) extraction and lipophilic organic solvent (such as petroleum ether, benzene, chloroform, ether, ethyl acetate, ethylene dichloride, propane, butane) extraction. Although the security of the water extraction method is better, the method has disadvantages such as longer extraction time and poor production efficiency while extracting at low temperature, and poor selectivity to the target extracts, serious degradation and high energy consumption while extracting at high temperature. For the organic solvent extraction method, it has hidden trouble on food safety due to organic solvent residue. Therefore, it is of great significance to develop efficient, safe and effective methods for extracting natural products, so as to effectively utilize biological resource, protect the ecological environment, save energy and reduce labor costs.

As researchers made their research work on natural products more widely and intensively, consumers know more about the physiological function natural products and have high demands for safety, producers pay more attention to the increasing consumer market of natural products, a number of extraction techniques such as microwave, ultrasound, high-voltage pulse electric field, supercritical fluids and ultra-high pressure technique and the like have been developed and applied. However, it is found that, the techniques of microwave, ultrasonic, high-voltage pulsed electric field cause significant degradation of the target in the extraction process; while supercritical fluid technology are mainly suitable for natural products with weak polarity and non-polarity, ultra-high pressure technology has strict requirements on equipments with the minimum processing stress of 100 MPa, resulting in high production cost.

EP 0 917 876 and US 6,326,504 are directed to the extraction of organic products from vegetable or animal matrices using a liquid solvent in the presence of one or various supercritical fluids and is based on inverting the proportions between supercritical fluid and conventional solvent, such that at a proportion of supercritical fluid is less than 50%.

### Summary of the Invention

The object of the present invention is to provide a novel method for extracting natural products, the method is non-toxic, harmless, and environment-friendly, it causes no degradation and oxidation to natural products, and it is suitable for large-scale industrial production, saves energy and requires low operation cost.

The method according to the present invention is mainly applied in the extraction of plant-derived natural products with polarity and some plant-derived natural products with medium polarity, such extracts are used in processing of agricultural products and food.

To achieve the objects stated above, the technical solution of the present invention provides a method for extracting plant-derived natural products with polarity or medium polarity, which comprises steps of shattering the plant-derived materials containing plant-derived natural products with polarity or medium polarity, adding water, raising the pressure to 5-50 MPa at the temperature of 40-90°C, and circularly feeding high-pressure carbon dioxide to conduct treatment for 1-60 min. In the invention, the extracts to be extracted are circularly treated with water as solvent in the presence of high-pressure carbon dioxide, and the security can be guaranteed because of using water as solvent. Meanwhile, high pressure carbon dioxide (HPCD) system is employed, in which high-pressure carbon dioxide comprises the forms of carbon dioxide molecule, carbonic acid, hydrogen ions in the dissociation state, bicarbonate ions and carbonate ions. In the process of extraction, high-pressure carbon dioxide exists in a dynamic mode and leads to several effects such as vortex, shear and mixing, which can improve the diffusion rate of the mass transfer, shorten the extraction time, and ensure the entire extraction process in a high-pressure, acidic and oxygen-free environment, and effectively avoid the degradation of the target extracts during the extraction process; when the pressure relief is conducted after extraction, the "explosion" effect of high-pressure carbon dioxide can further enhance damage to the cell structure of the extracted matrix, increasing the dissolution of intracellular materials to improve the yield. Meanwhile, high pressure carbon dioxide has bactericidal and enzyme-inactivating effect, which can inactivate and damage endogenous enzymes of natural products and kill microorganisms so as to extend the shelf life of natural products and ensure the subsequent large-scale, continuous purification and deep processing. The pressure of the extraction method is only ranging from 5-50 MPa, the requirements on equipments are lower than ultra-high pressure technology, and the method according to the present invention overcomes the deficiency of supercritical fluid technology in extraction of natural products with polarity or medium polarity. Therefore, the method according to the invention can expedite the extraction rate of the target extracts and increase yield so as to improve the production efficiency on the premise of guaranteeing the activity and safety of final products.

The extraction method above can be used to extract plant-derived natural products with polarity or medium polarity, such as anthocyanins, flavonoids, flavonols, tannins, polyphenols or glucosinolates. Of course, the substances with similar physical and chemical properties with said substances can also be extracted by the extraction method according to the invention.

The preferred temperature of extraction method above is in the range of 45-85°C.

According to the method above, the pressure is preferably in the range of 6-48 Mpa and the treatment time is preferably in the range of 10-50 min. The extraction method is carried out in the conventional reactor capable of circular ventilation, such as reaction vessel, reaction tank and the like, only provided that the reactor includes gas inlet and gas outlet, wherein the gas outlet is equipped with a filter, then a circulation pipe is provided behind the filter to connect to the gas inlet. A feed inlet is also essentially needed.

To further increase extraction efficiency, the volume ratio of the solid-liquid mixture (formed after adding water to plant-derived material) to the high-pressure carbon dioxide may be controlled in the range of 1:10-9:10, preferably 1:5-4:5. The ratio has great influence on the extraction efficiency, can increase the yield by around 15% and shorten the extraction time by around 40%. The pressure relief is carried out after completion of the treatment, and the extracts of natural products are discharged when the pressure is decreased to normal pressure.

The high-pressure carbon dioxide used in the method above can be recycled so as to reduce the release of carbon dioxide. This brings contamination to the environment and thus is environment-friendly and can meet the requirement of developing "low carbon" economy.

The above technical solution has the following advantages:
1) The method according to the present invention can be used not only for the extraction of polarity natural products such as anthocyanins, but also for the extraction of other natural products with polarity or medium polarity.
2) The high pressure carbon dioxide dynamically passes through the solid-liquid mixture and leads to effects such as vortex, shear and mixing, so as to improve the process of mass transfer, increase the speed and reduce the time.
3) The existence of high pressure carbon dioxide ensures the whole process in a high-pressure, acidic and oxygen-free environment and can effectively avoid degradation of the natural products during the extraction process.
4) During the pressure relief process, the "explosion" effect of high -pressure carbon dioxide can enhance the damage to the cellular structure of the extracted matrix, reduce the mass transfer resistance of target materials, and improve the extraction efficiency.
5) The HPCD technology not only can be used to extract the natural products, but also has the effects of sterilizing and inactivating enzyme for the extraction solution, so as to extend the shelf life of the natural products, and provide an important safeguard for large-scale and continuous production.
6) Carbon dioxide is a colorless, odorless, toxic gas, and it causes no adverse effects even if there is a residue, thus the safety of natural products is fully guaranteed.
7) Carbon dioxide can be circularly used in one extraction process, and recycled after several times of extraction, that makes HPCD technique become a green processing technology and meet the requirement of developing "low carbon" economy.

### Specific Modes for Carrying out the Invention

The invention is described below with reference to the following examples. These examples are provided for the purpose of illustration only and the invention should not be construed as being limited to these examples

### Example 1:

### Extraction of Anthocyanins

The plant matrix such as purple cabbage, which is rich in natural products anthocyanins, was shattered and placed in nylon bag, then put into a pre-heated reactor at the temperature of 40°C, water was added therein as extraction solvent, then the reactor was tightly closed. The pressure at the inlet was raised to 15 MPa, carbon dioxide passed through the solid-liquid mixture and spread to the exit of the reactor, after passing through the filter of the exit, the carbon dioxide entered the inlet again through the circulation pipe. The volume ratio of the solid-liquid mixture and the high pressure carbon dioxide is 1:5. The volume of high-pressure carbon dioxide was controlled by the volume of reactor and the volume of the purple cabbage solid-liquid mixture. After treated dynamically for 15min, the pressure was released, and the anthocyanin extract solution was discharged. Quantitative analysis, determination of microbial index and color evaluation were carried out on the anthocyanin extract solution.

The quantitative analysis of the yield of anthocyanins was carried out by pH differential method, the yield of anthocyanins obtained by the present method has been increased by about 20%, and the extraction time has been shortened by about 50% compared with the control trial. The color index analysis was carried out by using colorimeter, and the red values were all higher than that of control group. Moreover, this method effectively reduced the total number of bacteria in the extract solution.

The control trial is described as follows: the extraction was carried out under atmospheric pressure by the same method as in the above Example 1, except that the treatment was conducted under atmospheric pressure and the extraction time was defined as the time point when anthocyanins content in the extraction solution was kept constant, the other experimental conditions were completely the same as in the above extraction experiment.

### Example 2:

### Extraction of Anthocyanins

Except that the extraction was carried out at the temperature of 60°C under the pressure of 5 MPa, the volume ratio of the solid-liquid mixture and the high pressure carbon dioxide was 4:5, and the extraction time was 5 min, the rest specific operations were the same as in Example 1.

The yield of anthocyanins in purple cabbage obtained by the present method increased by about 10% compared with the control trial.

The definition of control trial was the same as described in Example 1.

### Example 3:

### Extraction of Anthocyanins

Except that the extraction was carried out at the temperature of 90°C under the pressure of 45 MPa, the volume ratio of the solid-liquid mixture and the high pressure carbon dioxide was 4:5, and the extraction time was 60 min, the rest specific operations were the same as in Example 1.

The yield of anthocyanins in purple cabbage obtained by the present method increased by about 8% compared with the control trial.

The definition of control trial was the same as described in Example 1.

### Example 4:

### Extraction of Flavonoids

The pulverized plant matrix such as hawthorn, which is rich in natural products flavonoids, was shattered and placed in nylon bag, except that the extraction was carried out at the temperature of 70°C under the pressure of 8 MPa, the volume ratio of the solid-liquid mixture and the high pressure carbon dioxide was 3:5, and the extraction time was 20 min, the rest specific operations were the same as in Example 1.

A quantitative analysis of the obtained flavonoids was carried out by sodium nitrite-aluminum nitrate colorimetric method. The yield of flavonoids obtained by the present method increased by about 7%, and the extraction time was shortened by about 30% compared with the control trial.

The definition of control trial is the same as described in Example 1.

### Example 5:

### Extraction of Flavonols

The plant matrix such as green tea, which is rich in natural products flavonols, was shattered and placed in nylon bag with the same operating parameters as in Example 4, and the rest operations were the same as in Example 1.

A quantitative analysis of the obtained flavonols was carried out by High Performance Liquid Chromatography. The yield of flavonols obtained by the present method increased by about 6% compared with the control trial.

The definition of control trial is the same as described in Example 1.

### Example 6:

### Extraction of Tannins

The plant matrix such as grape skin, which is rich in natural products tannins, was shattered and placed in nylon bag with the same operating parameters as in Example 4, and the rest operations were the same as in Example 1.

A quantitative analysis of the obtained tannins was carried out by spectrophotometry using potassium ferricyanide. The yield of tannins obtained by the present method increased by about 12%, and the extraction time was shortened by about 9% compared with the control trial.

The definition of control trial was the same as described in Example 1.

### Example 7:

### Extraction of Polyphenols

The plant matrix such as raspberry, which is rich in natural products polyphenols, was shattered and placed in nylon bag with the same operating parameters as in Example 4, and the rest operations were the same as in Example 1.

A quantitative analysis of total phenol contained in the obtained products is carried out by Folin-phenol method. The yield of total phenol obtained by the present method increased by about 10%, and the extraction time was shortened by about 40% compared with the control trial.

The definition of control trial was the same as described in Example 1.

### Example 8:

### Extraction of Glucosinolates

The plant matrix such as broccoli, which is rich in natural products glucosinolates, was shattered and placed in nylon bag, except that the extraction was carried out at the temperature of 60°C under the pressure of 7 MPa, the volume ratio of the solid-liquid mixture and high pressure carbon dioxide was 2:5, and the extraction time was 15 min, the rest specific operations were the same as in Example 1.

A quantitative analysis of glucosinolates was carried out by the method of sulfate ion precipitation. The yield of glucosinolates obtained by the present method increased by about 14%, and the extraction time was shortened by about 40% compared with the control trial.

The definition of control trial was the same as described in Example 1.

### Industrial Applicability

The extraction method according to the present invention can be readily carried out, while it has high extraction rate, low requirements on equipments, and no environment pollution during the extraction process. Since the extracted products are natural materials, it has wide range of applications and can be used in the fields of agricultural products processing and food processing; and the effects of sterilization and enzyme inactivation can extend the shelf life of the products, therefore the method according to the invention has high value in industrial application.

## Claims

1. A method for extracting plant-derived natural products with polarity or medium polarity by circular treatment using water as extraction solvent in the presence of high-pressure carbon dioxide , comprising steps of shattering the plant-derived materials containing plant-derived natural products with polarity or medium polarity, adding water, raising the pressure to 5-50 MPa at the temperature of 40-90°C, and circularly feeding high-pressure carbon dioxide to conduct treatment for 1-60 min, **characterized in that** the volume ratio of the solid-liquid mixture formed after adding water to the plant-derived materials and the high-pressure carbon dioxide is 1:10-9:10, wherein the volume of high-pressure carbon dioxide is controlled by the reactor volume and the volume of the solid-liquid mixture.

2. The method according to claim 1, wherein the plant-derived natural products with polarity or medium polarity are selected from the group consisting of anthocyanins, flavonoids, flavonols, tannins, polyphenols and glucosinolates.

3. The method according to claim 1 or 2, wherein the temperature is in the range of 45-85°C.

4. The method according to any of the previous claims, wherein the pressure is in the range of 6-48 MPa.

5. The method according to any of the previous claims, wherein the treatment time is in the range of 10-50 min.

6. The method according to any of the previous claims, wherein the volume ratio of the solid-liquid mixtures formed after adding water to the plant-derived materials and the high-pressure carbon dioxide is 1:5-4:5.

## Patentansprüche

1. Verfahren zum Extrahieren von von Pflanzen abgeleiteten natürlichen Produkten mit Polarität oder mittlerer Polarität durch kreisförmige Behandlung unter Verwendung von Wasser als Extraktionslösungsmittel in Gegenwart von Hochdruck-Kohlendioxid, umfassend Schritte von Zerbrechen der von Pflanzen abgeleiteten Materialien, enthaltend von Pflanzen abgeleitete natürliche Produkte mit Polarität oder mittlerer Polarität, Hinzufügen von Wasser, Erhöhen des Drucks auf 5-50 MPa bei der Temperatur von 40-90°C und kreisförmiges Zuführen von Hochdruck-Kohlendioxid zur Durchführung der Behandlung für 1-60 Min., **dadurch gekennzeichnet, dass** das Volumenverhältnis des Feststoff-Flüssigkeitsgemischs, gebildet nach Hinzufügen von Wasser zu den von Pflanzen abgeleiteten Materialien und des Hochdruck-Kohlendioxids 1:10-9:10 ist, wobei das Volumen des Hochdruck-Kohlendioxids durch das Reaktorvolumen und das Volumen des Feststoff-Flüssigkeitsgemischs gesteuert wird.

2. Verfahren nach Anspruch 1, wobei die von Pflanzen abgeleiteten natürlichen Produkte mit Polarität oder mittlerer Polarität ausgewählt sind aus der Gruppe bestehend aus Anthocyaninen, Flavonoiden, Flavonolen, Tanninen, Polyphenolen und Glucosinolaten.

3. Verfahren nach Anspruch 1 oder 2, wobei die Temperatur in dem Bereich von 45-85°C liegt.

4. Verfahren nach einem der vorhergehenden Ansprüche, wobei der Druck in dem Bereich von 6-48 MPa liegt.

5. Verfahren nach einem der vorhergehenden Ansprüche, wobei die Behandlungszeit in dem Bereich von 10-50 Min. liegt.

6. Verfahren nach einem der vorhergehenden Ansprüche, wobei das Volumenverhältnis der Feststoff-Flüssigkeitsgemische, gebildet nach Hinzufügen von Wasser zu den von Pflanzen abgeleiteten Materialien und des Hochdruck-Kohlendioxids 1:5-4:5 ist.

## Revendications

1. Un procédé d'extraction de produits naturels d'origine végétale présentant une polarité ou une polarité moyenne par traitement circulaire, utilisant de l'eau en tant que solvant d'extraction, en présence de dioxyde de carbone sous pression élevée, comprenant
- des étapes d'égrenage des produits naturels d'origine végétale, présentant une polarité ou une polarité moyenne,
- l'addition d'eau,
- l'augmentation de la pression au-dessus de 5-50 MPa à la température de 40 à 90°C, et
- l'alimentation circulaire du dioxyde de carbone à haute pression pour procéder à un traitement pendant 1 à 60 min,
**caractérisé en ce que** le rapport volumique du mélange solide-liquide formé après addition d'eau aux matériaux d'origine végétale et au dioxyde de carbone sous pression élevée est de 1/10 à 9/10,
et **en ce que** le volume du dioxyde de carbone sous pression élevée est contrôlé par le volume du réacteur et le volume du mélange solide-liquide.

2. Le procédé selon la revendication 1, dans lequel les produits naturels d'origine végétale présentant une polarité ou une polarité moyenne sont choisis dans le groupe constitué par les anthocyanines, les flavonoïdes, les flavonols, les tannins, les polyphénols et les glucosinolates.

3. Le procédé selon la revendication 1 ou 2, dans lequel la température est comprise dans l'intervalle de 45-85° C.

4. Le procédé selon l'une quelconque des revendications précédentes, dans lequel la pression est comprise dans l'intervalle de 6-48 MPa.

5. Le procédé selon l'une quelconque des revendications précédentes, dans lequel la durée de traitement est comprise dans l'intervalle de 10 à 50 min.

6. Le procédé selon l'une quelconque des revendications précédentes, dans lequel le rapport volumique des mélanges solides-liquides formés après addition d'eau aux matières d'origine végétale et du dioxyde de carbone à haute pression est de 1/5-4/5.
